# EUROPÄISCHE PATENTSCHRIFT

(11) **EP 1 418 894 B1**
(45) Veröffentlichungstag und Bekanntmachung des Hinweises auf die Patenterteilung: **24.01.2007**
(21) Anmeldenummer: 02762336.2
(22) Anmeldetag: 10.07.2002
(51) Int. Cl.: A61K 9/70, A61K 31/4468

(54) **TRANSDERMALES THERAPEUTISCHES SYSTEM MIT FENTANYL**
TRANSDERMAL THERAPEUTIC SYSTEM WITH FENTANYL
SYSTEME THERAPEUTIQUE TRANSDERMIQUE CONTENANT DU FENTANYLE

(30) Priorität: 24.08.2001 DE 10141650
(43) Veröffentlichungstag der Anmeldung: 19.05.2004
(73) Patentinhaber: LTS LOHMANN Therapie-Systeme AG, 56626 Andernach (DE)
(72) Erfinder: MÜLLER, Walter, 56626 Andernach (DE); HILLE, Thomas, 56567 Neuwied (DE)
(74) Vertreter: Isenbruck, Günter
(86) Internationale Anmeldenummer: PCT/EP2002/007664
(87) Internationale Veröffentlichungsnummer: WO 2003/018075

(56) Entgegenhaltungen:
- WO-A2-02/24157
- US-B1- 6 210 705
- ROY S D ET AL: "CONTROLLED TRANSDERMAL DELIVERY OF FENTANYL: CHARACTERIZATIONS OF PRESSURE-SENSITIVE ADHESIVES FOR MATRIX PATCH DESIGN" JOURNAL OF PHARMACEUTICAL SCIENCES, AMERICAN PHARMACEUTICAL ASSOCIATION. WASHINGTON, US, Bd. 85, Nr. 5, 1. Mai 1996 (1996-05-01), Seiten 491-495, XP000583527 ISSN: 0022-3549
- PATENT ABSTRACTS OF JAPAN vol. 015, no. 001 (C-0793), 7. Januar 1991 (1991-01-07) & JP 02 255611 A (NITTO DENKO CORP), 16. Oktober 1990 (1990-10-16)

## Beschreibung

Fentanyl ist ein außerordentlich wirksames Analgetikum. Das Erfordernis der nur geringen Dosierung und seine physiko-chemischen Eigenschaften wie der n-Octanol-Wasser-Verteilungskoeffizient, Schmelzpunkt und das Molekulargewicht machen die transdermale Zufuhr von Fentanyl in wirksamer Menge möglich und seine pharmakokinetischen Eigenschaften wie die schnelle Metabolisierung und der relativ enge therapeutische Index die transdermale Zufuhr wünschenswert.

In der Tat ist seit einigen Jahren ein TTS mit Fentanyl als Wirkstoff auf dem Markt. Dieses System ist ein sogenanntes Reservoirsystem. Unter einem Reservoirsystem wird dabei ein System verstanden, das den Wirkstoff in einer flüssigen oder gelförmigen Zubereitung in einem aus einer undurchlässigen Folie, die als Rückschicht dient, und einer wirkstoffdurchlässigen Membran geformten Beutel enthält, wobei die Membran zusätzlich mit einer Kleberschicht zur Befestigung des Systems auf der Haut versehen ist. In diesem speziellen Fall ist Fentanyl in einem Gemisch aus Ethanol und Wasser gelöst. Weitere Einzelheiten dieses Systems können der US-Patentschrift 4,588,580 bzw. der DE-PS 35 26 339 entnommen werden, die beide eine detaillierte Beschreibung enthalten.

Reservoirsysteme haben allerdings den Nachteil, daß im Falle einer Undichtigkeit des Reservoirbeutels die wirkstoffhaltige Reservoirfüllung großflächig mit der Haut in Kontakt kommt und der Wirkstoff in zu hohen Dosen resorbiert wird. Dies ist speziell bei Fentanyl sehr gefährlich, da eine Überdosierung sehr schnell zu Atemdepression und damit tödlichen Zwischenfällen führt. Mehrere solcher tödlichen bzw. fast tödlichen Zwischenfälle sind beschrieben in *Clinical Pharmacokinet.* **2000**, 38(1), 59-89.

Aufgabe dieser Erfindung war es nun ein transdermales therapeutisches System mit Fentanyl bereitzustellen, das dem Benutzer eine erhöhte Sicherheit gegen eine versehentliche Aufnahme von Überdosen bietet.

Dies gelingt erfindungsgemäß durch das im Anspruch 1 definierte TTS. Dabei wird statt des Reservoirsystems ein X Matrixsystem eingesetzt bei dem Fentanyl direkt in ein selbstklebendes Polyacrylat eingearbeitet wird und damit selbst bei einer Beschädigung des Systems nicht in einer größeren als durch das TTS gegebenen Fläche mit der Haut in Kontakt kommen kann. Fentanyl ist in einem solchen System im allgemeinen ganz, jedoch zu mindestens 80 % in diesem Polymer molekulardispers gelöst, wobei die Sättigungslöslichkeit des Fentanyls im Polymer zwischen 3 und 20 Gewichtsprozenten liegt. Weiterhin hat es sich überraschenderweise gezeigt, daß bei der Verwendung von Polyacrylatklebern für die Herstellung von TTS mit Fentanyl nur Kleber ohne freie Carboxylgruppen geeignet sind.

Solche Matrixsysteme bestehen im einfachsten Fall aus einer Rückschicht, die für den Wirkstoff undurchlässig ist, einer selbstklebenden wirkstoffhaltigen Schicht und einer vor Gebrauch zu entfernenden Schutzschicht. In komplizierteren Ausführungen solcher Systeme schließt sich zusätzlich noch eine die Wirkstoff abgabe steuernde Membran an, die normalerweise noch mit einer Kleberschicht zur Befestigung des Systems auf der Haut versehen ist.

Die Fentanyl haltigen Schichten eines solchen Matrixsystems gemäß dieser Erfindung bestehen aus den in Anspruch 1 definierten Polyacrylaten. Da freie funktionelle Gruppen die Sättigungslöslichkeit von Fentanyl in Polyacrylatklebern über den bevorzugten Bereich erhöhen, erfindungsgemäß solche Polyacrylatkleber eingesetzt die über keine freien funktionellen Gruppen verfügen und lediglich aus Estern der Acryl- und/oder Methaycrylsäure und gegebenenfalls Vinylacetat hergestellt werden. Allerdings können bei der Klebersynthese Monomere mit freien Hydroxylgruppen wie 2-Hydroxyethylacrylat oder 2-Hydroxyethylmethacrylat bis zu einem Anteil von 20 Gew.-% toleriert werden. Polyacrylate werden durch radikalische Polymerisation unter Verwendung von Acryl- und/oder Methacrylsäure estern hergestellt, diese sind Ester von Alkoholen mit 1 bis 8 C-Atomen, die gegebenenfalls eine Hydroxylgruppe enthalten, wie 2-Ethylhexylacrylat, n-Octylacrylat, Propylacrylat, n- oder iso-Butylacrylat, und 2-Hydroxyethylacrylat bzw. die entsprechenden Methacrylate. Zusätzlich kann auch Vinylacetat mitverwendet werden und zwar in Mengen von bis zu 50 Gew.-%. Die so hergestellten Polymere werden auch als statistische Copolymere bezeichnet, da allein die Mengenverteilung der eingesetzten Monomeren und der Zufall über die Zusammensetzung der Polymerketten entscheidet.

Enthalten die Polymere freie Hydroxylgruppen, besteht die Möglichkeit, die Polymerketten zusätzlich durch mehrwertige Kationen wie Al³⁺ oder Ti⁴⁺ oder reaktive Substanzen wie Melamin zu vernetzen. Man macht von dieser Möglichkeit Gebrauch um das Molekulargewicht zu erhöhen und damit die Kohäsion der Polymere zu verbessern. Die Möglichkeit der Quervemetzung von Polyacrylaten, insbesondere von Polyacrylatklebern, ist besonders wertvoll, wenn die weichmachende Wirkung des in den Polymeren gelösten Fentanyls bzw. die weichmachende Wirkung von anderen Hilfsstoffen kompensiert werden muß. Der Kleber wird gewöhnlich in Form einer Lösung verwendet. Als Lösungsmittel dienen z. B. Ethylacetat, Hexan oder Heptan, Ethanol oder deren Mischungen. Diese werden während der Herstellung des TTS entfernt.

In Tabelle 1 sind die Ergebnisse von Permeationsstudien gezeigt, die mit einem Kleber mit und einem Kleber ohne freie Carboxylgruppen (jedoch mit Hydroxylgruppen) erreicht worden sind. In beide Kleber wurde Fentanyl in einer Konzentration von 5 Gewichtsprozenten eingearbeitet. Die Permeationsstudie wurde mittels dem Fachmann bekannter Franz-Diffusionszellen und Verwendung von menschlicher Haut durchgeführt.

**Tabelle 1: Ergebnisse von Permeationsstudien mit Klebern mit und ohne freie Carboxylgruppen**

| Formulierung | Kumulierte Menge an permeiertem Fentanyl [µg/cm²] Mittelwert von n = 3 * | | | | |
|---|---|---|---|---|---|
| | 4 h | 8 h | 24 h | 48 h | 72 h |
| 1 | 0,00 | 0,00 | 0,44 | 1,71 | 3,51 |
| 2 | 0,0 | 0,2 | 4,0 | 14,7 | 28,24 |

| | | | | | |
|---|---|---|---|---|---|
| * verwendete Haut: weibl. Unterleibshaut | | | | | |

- Formul. 1:: Polyacrylatkleber mit 4,8 Gew.-% freien Carboxylgruppen
- Formul. 2:: neutraler Polyacrylatkleber ohne freie Carboxylgruppen aber mit 5,2 Gew.-% freien Hydroxylgruppen

Die Ergebnisse zeigen, daß ein neutraler Kleber ohne freie Carboxylgruppen einem carboxylgruppenhaltigen Kleber bezüglich der erreichbaren Permeationsraten deutlich überlegen ist.

Eine wichtige Eigenschaft jedes wirkstoffhaltigen Polymers in der TTS-Technologie ist die Sättigungslöslichkeit des gewählten Polymers für den jeweiligen Wirkstoff. Dieser Parameter ist deshalb wichtig, weil die thermodynamische Aktivität des Wirkstoffs in der Matrix nicht von der absolut gelösten Menge des Wirkstoffs, sondern vielmehr vom Verhältnis der tatsächlichen Konzentration zu der Sättigungskonzentration abhängt. Da sich der Wirkstoff bei der Applikation des TTS auf die Haut in die Haut verteilen muß und sich dabei nicht Konzentrationen, sondern Aktivitäten angleichen, ist es zum Erreichen einer möglichst hohen Permeationsrate wichtig, die thermodynamische Aktivität des Wirkstoffs im TTS möglichst hoch zu wählen. Dies bedeutet, daß die Löslichkeit des Wirkstoffs in den wirkstoffhaltigen Teilen des TTS nicht zu hoch sein darf, da ansonsten die Wirkstoffkonzentration im TTS recht hoch sein muß um eine genügend hohe thermodynamische Aktivität zu erreichen. Dies ist unvorteilhaft, wenn der Wirkstoff in der hohen Konzentration die physikalischen Eigenschaften der wirkstoffhaltigen Teile des Systems nachteilig beeinflußt und/oder der Wirkstoff sehr teuer ist. Im Falle des Fentanyl treffen beide Gründe zu, wobei zusätzlich noch in Betracht zu ziehen ist, daß Fentanyl zu den Betäubungsmitteln zählt und es allein schon deshalb wünschenswert ist, möglichst wenig Fentanyl in das TTS einzuarbeiten bzw. die Fentanyl ausnutzung, d.h. das Verhältnis von während der Tragezeit des TTS abgegebenem Fentanyl zum Gehalt des ungetragenen TTS möglichst groß zu gestalten.

Unter dem Gesichtspunkt sollte die Sättigungslöslichkeit der Fentanyl haltigen Schichten für ein Dreitage-TTS nicht unter 3 Gewichtsprozenten und nicht über 20 Gewichtsprozenten liegen. Bei höheren Sättigungslöslichkeiten wird selbst bei einer hohen spezifischen Permeationsrate die Wirkstoffausnutzung zu schlecht, und das TTS ist aus kommerziellen Gründen wegen des teuren Wirkstoffs nicht gut verkäuflich. Bevorzugt liegt aus diesen Gründen die Sättigungslöslichkeit zwischen 4 und 12 und besonders bevorzugt zwischen 5 und 10 Gewichtsprozenten.

Die Sättigungslöslichkeit von Fentanyl kann zusätzlich reduziert werden durch den Zusatz von Substanzen, die keine guten Löseeigenschaften für Fentanyl haben. Solche Substanzen sind z.B. flüssige Kohlenwasserstoffe wie Dioctylcyclohexan, flüssiges Paraffin, Kohlenwasserstoffharze wie Polyterpene, insbesondere Polypinen oder polare Substanzen wie Glycerin, Di- und Triglycerin oder Polyethylenglykole, z. B. mit einem Molgewicht von 200 bis 1000. Diese Substanzen können mit dem Polyacrylatkleber eine homogene Mischung bilden oder aber als eine gesonderte Phase darin enthalten sein. Speziell Glycerin und seine Derivate liegen schon bei geringen Konzentrationen in der Matrix als gesonderte Phase, z. B. in Form von Tröpfchen vor. Durch die Zugabe solcher Substanzen kann insbesondere auch die höhere Sättigungslöslichkeit in Klebern mit freien Hydroxylgruppen kompensiert werden.

Tabelle 2 enthält einige Angaben zu den Sättigungslöslichkeiten von Fentanyl, in einigen dieser Substanzen.

Tabelle 2: Sättigungslöslichkeiten von Fentanyl in löslichkeitsvermindernden Zusätzen

| **Substanz** | **Sättigungslöslichkeit [Gew.-%]** |
|---|---|
| Polyethylenglykol 400 | 7,5 |
| Glycerin | < 1,5 |
| Diglycerin | < 1,5 |
| Dioctylcyclohexan | < 1,9 |
| Paraffin, flüssig | < 1,5 |

Der Einfluß von solchen Zusätzen läßt sich anhand von vergleichenden Permeationsstudien erkennen.

In Tabelle 3 sind die Ergebnisse von Permeationsstudien mit Matrices auf Basis eines neutralen Polyacrylatklebers mit freien Hydroxylgruppen mit und ohne solche Zusätze sowie eines Polyacrylatklebers ohne andere freie funktionelle Gruppen gegenübergestellt. Alle Formulierungen enthalten Fentanyl in einer Konzentration von 5 Gew. %.

**Tabelle 3. Vergleichende Permeationsstudien mit Formulierungen mit und ohne löslichkeitsvermindernde Zusätze**

| Formulierung | Kumulierte Menge an permeiertem Fentanyl [µg/cm²] Mittelwert von n = 3 * | | | | |
|---|---|---|---|---|---|
| | 4 h | 8 h | 24 h | 48 h | 72 h |
| 2 | 0,00 | 0,23 | 7,89 | 32,82 | 64,17 |
| 3 | 0,798 | 4,46 | 29,6 | 68,9 | 103,1 |
| 4 | 0,805 | 4,87 | 32,6 | 74,7 | 113,2 |

| | | | | | |
|---|---|---|---|---|---|
| • Haut: menschliche Epidermis, weibliche Brusthaut | | | | | |

| | | |
|---|---|---|
| Formul. 2: | 5 Gew. % Fentanyl in einem neutralen Polyacrylatkleber mit 5,2 % freien Hydroxylgruppen | |
| Formul. 3: | Fentanyl | 5,0 % |
| | Polyacrylatkleber, neutral mit 5,2% freien Hydroxylgruppen | 55,0 % |
| | Polypinen | 15,0 % |
| | Glycerin | 10,0 % |
| | Dioctylcyclohexan | 15,0 % |
| Formul. 4: | 5 Gew. % Fentanyl in einem Polyacrylatkleber ohne freie funktionelle Gruppen | |

Die Ergebnisse der Permeationsstudie zeigen, daß die Permeationsrate durch den Zusatz von die Löslichkeit des Wirkstoffs in der Matrix reduzierenden Substanzen signifikant verbessert werden kann. In etwa die gleichen Ergebnisse erreicht man durch die Verwendung eines Klebers ohne freie funktionelle Gruppen, der auch ohne Zusätze über eine geringe Lösekapazität für Fentanyl verfügt.

Aus den Permeationsdaten lassen sich für verschiedene TTS-Stärken die jeweiligen TTS-Größen berechnen. Die Ergebnisse sind in Tabelle 4 gelistet.

**Tabelle 4: Aus Permeationsdaten errechnete TTS-Größen**

| **Abgaberate** | **berechnete Flächengrößen [cm²]** | | | | |
|---|---|---|---|---|---|
| | **Form. 1** | **Form. 2 *** | **Form. 2 **** | **Form. 3** | **Form. 4** |
| **25 µm/ h** | 513 | 63,7 | 28,1 | 17,45 | 15,9 |
| **50 µm/ h** | 1026 | 127,4 | 56,2 | 34,9 | 31,8 |
| **75 µm/ h** | 1539 | 191,1 | 84,3 | 52,35 | 47,7 |
| **100 µm/ h** | 2052 | 254,8 | 112,4 | 69,8 | 63,6 |

| | | | | | |
|---|---|---|---|---|---|
| * berechnet auf Basis der Permeationsdaten aus Tabelle 1 ** berechnet auf Basis der Permeationsdaten aus Tabelle 2 | | | | | |

Das Ergebnis der Berechnung zeigt, daß carboxylgruppenhaltige Kleber bei einer Fentanylkonzentration von 5 % auch bei der niedrigsten Dosierung zu für den praktischen Gebrauch zu großen TTS führen. Bei den hydroxylgruppenhaltigen Klebern berechnen sich zwar auch recht große TTS, allerdings besteht hier durch die Erhöhung der Fentanylkonzentration die Möglichkeit, bei nicht zu hohen Konzentrationen, d.h. höchstens 20 %, zu TTS mit einer für den praktischen Gebrauch geeigneten Größe zu kommen. Vereinfacht kann dabei angenommen werden, daß die thermodynamische Aktivität und damit auch die Permeationsraten linear von der Konzentration abhängen, solange Fentanyl vollständig gelöst vorliegt.

Durch Verwendung von die Löslichkeit senkenden Hilfsstoffen in Formulierungen mit hydroxylgruppenhaltigen Polyacrylatklebern bzw. durch die Verwendung von Polyacrylatklebern ohne freie funktionelle Gruppen erhält man schon bei einer Fentanylkonzentration von 5,0 % TTS, die selbst in der höchsten Dosierung von 100 µg/ h eine akzeptable Größe aufweisen. Natürlich bietet sich auch hier die Möglichkeit, durch eine Erhöhung der Fentanylkonzentration die Systemfläche weiter zu verkleinern.

Fentanyl hat einen engen therapeutischen Index. Dies bedeutet, daß zur Wirkung einerseits ein gewisser Schwellenwert, der bezüglich der Plasmakonzentration überschritten sein muß, andererseits bei höheren Konzentrationen schnell unakzeptable Nebenwirkungen auftreten. Es ist deshalb vorteilhaft, wenn das System zusätzlich eine Steuermembran enthält und damit den Wirkstofffluß durch die Haut unabhängig von der individuellen Hautbeschaffenheit auf einen Maximalwert begrenzt. Solche Membranen bestehen bevorzugt aus einem Copolymer aus Ethylen und Vinylacetat (EVA-Polymer) oder sind mikroporöse Folien auf der Basis von Polyethylen oder Polypropylen. Derartige Membranen gehören zum Stand der Technik. Im Falle der-EVA-Polymere hängt dieWirkstoffdurchtässigkeitvon dem Anteil des Vinylacetats und der Dicke der Membran ab. Gebräuchlich sind Membranen mit einem VA-Gehalt zwischen 2 und 25 Gewichtsprozenten und einer Dicke zwischen 25 und 100 µm, vorzugsweise zwischen 40 und 100µm, wobei es bezüglich des Vinylacetat-Gehalts und der Dicke praktisch kaum Begrenzungen gibt. Für die jeweilige Formulierung müssen beide Parameter entsprechend gewählt werden, um die Begrenzung auf den gewünschten Maximalfluß aus dem TTS zu gewährleisten. Bei den mikroporösen Membranen erfolgt der Stofftransport nicht durch das Polymer, sondern lediglich durch die sich in diesen Membranen befindlichen Poren. Die Anzahl und Größe der Poren bestimmt dabei die maximale Abgaberate des TTS.

Üblicherweise sind solche Membranen mit einem Kleberfilm zur Befestigung des TTS auf der Haut versehen. Besonders geeignet für Fentanyl sind Kleberfilme auf Basis von selbstklebenden Polyacrylaten oder Fentanyl selbstklebenden Polysiloxanen. Der Vorteil von Polysiloxanen ist dabei, daß in diesen Polymerem sehr schlecht löslich ist und deshalb die Wirkstoffbeladung des TTS durch die Verwendung eines zusätzlichen Kleberfilms nicht unnötig gesteigert werden muß. Derartige Kleberfilme können jedoch auch bei Systemen angewendet werden, die keine Membranen, aber Matrixschichten mit geringerer Klebkraft enthalten.

Wie bei jedem TTS gibt es natürlich auch hier die Möglichkeit, die Barriereeigenschaften des menschlichen Stratum Corneum durch den Einsatz von permeationsfördernden Stoffen zu reduzieren. Solche Substanzen sind z.B. Fettsäuren, Fettalkohole, Fettsäureester, Ester des Glycerins mit mittel- bzw. langkettigen Fettsäuren und Glycole wie 1,2-Propandiol. Es können dabei alle Substanzen eingesetzt werden, die physiologisch unbedenklich und mit Fentanyl und den anderen Hilfsstoffen verträglich sind.

Zusammenfassend ist festzustellen, das Matrixsysteme im Sinne dieser Erfindung befriedigende bis gute Permeationsraten zeigen und auch die Herstellung von TTS mit einer akzeptablen Größe ermöglichen. Gleichzeitig ist eine Gefährdung des Patienten durch eine zu hohe Fentanyl aufnahme infolge einer Undichtigkeit unmöglich. Insgesamt stellen damit Matrixsysteme auf Basis von Polyacrylatklebern im Sinne dieser Erfindung für Fentanyl bezüglich der Patientensicherheit einen bedeutenden Fortschritt gegenüber dem bekannten Stand der Technik dar.

### Beispiele:

### Beispiel 1 (Formulierung 1, 2, 4)

Fentanyl (freie Base) wird in der Lösung des Klebers im Heptan/Ethylacetat gelöst. Die Menge an Fentanyl wird dabei so berechnet, daß sich, bezogen auf den Feststoffgehalt der Kleberlösung, eine Konzentration von 5,0 % ergibt. Die resultierende Masse wird mit einem Rakel auf eine silikonisierte Polyesterfolie vor Gebrauch zu entfernende Schutzschicht, in einer Dicke beschichtet, daß sich nach dem Entfernen der Lösemittel ein Gewicht der Beschichtung von ca. 80 g/ m² ergibt. Nach dem Entfernen der Lösemittel wird der getrocknete Film mit einer dünnen Polyesterfolie (Rückschicht des TTS) kaschiert, und aus dem Gesamtlaminat werden die fertigen TTS ausgestanzt.

### Beispiel 2 (Formulierung 3):

5,0 g Fentanyl, 15,0 g Polypinen, 10,0 g Glycerin, 15,0 g Dioctvlcyclohexan und 110 g der Kleberlosung mit einem Feststoffgehalt von 50,0 % werden zusammengegeben und bis zum Auflösen des Fentanyls gerührt. Die resultierende Masse wird mit einem Rakel auf eine silikonisierte Polyesterfolie (vor Gebrauch zu entfernende Schutzschicht) in einer Dicke beschichtet, daß sich nach dem Entfernen der Lösemittel ein Gewicht der Beschichtung von ca. 80 g/m² ergibt. Nach dem Entfernen der Lösemittel wird der getrocknete Film mit einer dünnen Polyesterfolie (Rückschicht des TTS) kaschiert und aus dem Gesamtlaminat werden die fertigen TTS ausgestanzt.

## Patentansprüche

1. Transdermales Therapeutisches System (TTS) bestehend aus einer wirkstoffundurchlässigen Rückschicht, zumindest einer Fentanyl enthaltenden Matrixschicht auf Basis von Polyacrylat und einer vor Gebrauch zu entfernenden Schutzschicht, **dadurch gekennzeichnet, dass** das Polyacrylat selbstklebend, frei von Carboxylgruppen ist, lediglich aus monomeren Estern von Alkoholen mit 1 bis 8 C-Atomen, die gegebenenfalls eine Hydroxylgruppe enthalten, und Acrylsäure und/oder Methacrylsäure und gegebenenfalls aus bis zu 50 Gew.-% Vinylacetat hergestellt wird, für Fentanyl eine Sättigungslöslichkeit zwischen 3 und 20 Gewichtsprozenten aufweist, und dass die wirkstoffhaltigen Schichten mindestens 5 Gewichtsprozent des eingearbeiteten Fentanyls enthalten, von dem mindestens 80 Gewichtsprozent in molekulardispers gelöster Form vorliegen.

2. TTS gemäß Anspruch 1, **dadurch gekennzeichnet, dass** das Polyacrylat für Fentanyl eine Sättigungslöslichkeit zwischen 4 und 12 Gewichtsprozenten aufweist.

3. TTS gemäß Anspruch 2, **dadurch gekennzeichnet, dass** das Polyacrylat für Fentanyl eine Sättigungslöslichkeit zwischen 5 und 10 Gewichtsprozenten aufweist.

4. TTS gemäß Anspruch 1, **dadurch gekennzeichnet, dass** das Polyacrylat keine Hydroxylgruppen enthält.

5. TTS gemäß einem der Ansprüche 1 bis 3, **dadurch gekennzeichnet, dass** das dem Polyacrylat zugrunde liegende Monomerengemisch bis zu 20 Gew.-% 2-Hydroxyethylacrylat und/oder -methacrylat als Monomere mit freien Hydroxylgruppen enthält.

6. TTS gemäß einem der Ansprüche 1 bis 3 und 5, **dadurch gekennzeichnet, dass** die Polymerketten im Polyacrylat mit freien Hydroxylgruppen durch mehrwertige Kationen oder reaktive Substanzen vernetzt sind.

7. TTS gemäß Anspruch 6, **dadurch gekennzeichnet, dass** die Vernetzung durch A/³⁺, Ti⁴⁺ oder Melamin erfolgt ist.

8. TTS gemäß einem der Ansprüche 1 bis 7, **dadurch gekennzeichnet, dass** es als weitere Schicht zusätzlich eine Steuermembran enthält.

9. TTS gemäß Anspruch 8, **dadurch gekennzeichnet, dass** es zusätzlich eine sich hautwärts auf der Steuermembran befindende selbstklebende Schicht zur Befestigung auf der Haut enthält.

10. TTS gemäß Anspruch 8 oder 9, **dadurch gekennzeichnet, dass** die Steuermembran aus einem Ethylen-Vinylacetat-Copolymer, zweckmäßig mit einem Vinylacetatanteil von bis zu 25 Gew.-%, oder einer mikroporösen Folie auf Basis von Polyethylen oder Polypropylen besteht und zweckmäßig eine Dicke zwischen 25 und 100, vorzugsweise zwischen 40 und 100 µm, aufweist.

11. TTS gemäß einem der Ansprüche 1 bis 10, **dadurch gekennzeichnet, dass** die Fentanylhaltigen Schichten zusätzlich die Permeationsrate durch menschliche Haut verbessernde Substanzen enthalten, insbesondere Glykole und/oder solche, die zur Gruppe der Fettsäuren, Fettsäureester, Fettalkohole oder Glycerinester gehören.

12. TTS gemäß einem der Ansprüche 1 bis 11, **dadurch gekennzeichnet, dass** die Fentanylhaltigen Schichten Substanzen enthalten, die die Löslichkeit des Fentanyls in diesen Schichten erniedrigen.

13. TTS gemäß Anspruch 12, **dadurch gekennzeichnet, dass** die die Löslichkeit erniedrigenden Substanzen bei Raumtemperatur flüssige Kohlenwasserstoffe, insbesondere Dioctylcyclohexan oder flüssiges Paraffin, Kohlenwasserstoffharze, insbesondere Polypinenharze oder Polyethylenglykol oder Glycerin, sind.

## Claims

1. A transdermal therapeutic system (TTS) consisting of a active compound-impermeable back layer, at least one matrix layer based on polyacrylate and comprising fentanyl, and a protective film to be removed before use, **characterized in that** the polyacrylate is self-adhesive and free of carboxyl groups, and is synthesized only from monomeric esters of alcohols comprising 1 to 8 C atoms, the alcohols having optionally a hydroxyl group, and of acrylic and/or methacrylic acid and, if appropriate, vinyl acetate in an amount of up to 50 percent by weight, has a saturation solubility for fentanyl of between 3 and 20 percent by weight, and **in that** the active compound-containing layers contain at least 5 percent by weight of the incorporated fentanyl, 80 percent by weight thereof being in molecularly disperse dissolved form.

2. The TTS as claimed in claim 1, **characterized in that** the polyacrylate has a saturation solubility of between 4 and 12 percent by weight for fentanyl.

3. The TTS as claimed in claim 2, **characterized in that** the polyacrylate has a saturation solubility of between 5 and 10 percent by weight for fentanyl.

4. The TTS as claimed in claim 1, **characterized in that** the polyacrylate does not contain hydroxyl groups.

5. The TTS as claimed in any of claims 1 to 3, **characterized in that** the monomer mixture on which the polyacrylate is based contains up to 20% by weight 2-hydroxyethyl acrylate and/or methacrylate as monomers having free hydroxyl groups.

6. The TTS as claimed in any of claims 1 to 3 and 5, **characterized in that** the polymer chains of the polyacrylate having free hydroxyl groups are cross-linked by polyvalent cations or reactive substances.

7. The TTS as claimed in claim 6, **characterized in that** the crosslinking has been conducted by Al³⁺, Ti⁴⁺ or melamine.

8. The TTS as claimed in any of claims 1 to 7, **characterized in that** it additionally contains a control membrane as a further layer.

9. The TTS as claimed in claim 8, **characterized in that** it additionally contains a self-adhesive layer situated toward the skin on the control membrane for fixing to the skin.

10. The TTS as claimed in claim 8 or 9, **characterized in that** the control membrane consists of an ethylene/vinyl acetate copolymer, expediently having a vinyl acetate content of up to 25% by weight, or of a microporous film based on polyethylene or polypropylene and expediently has a thickness of between 25 and 100, preferably between 40 and 100 µm.

11. The TTS as claimed in any of claims 1 to 10, **characterized in that** the fentanyl containing layers additionally contain substances improving the permeation rate through human skin, in particular glycols and/or those which belong to the group consisting of the fatty acids, fatty acid esters, fatty alcohols or glycerol esters.

12. The TTS as claimed in any of claims 1 to 11, **characterized in that** the fentanyl containing layers contain substances which lower the solubility of fentanyl in these layers.

13. The TTS as claimed in claim 12, **characterized in that** the substances lowering the solubility are hydrocarbons which are liquid at room temperature, preferably dioctylcyclohexane or liquid paraffin, hydrocarbons resins, preferably polypinene resins, or polyethylene glycol or glycerol.

## Revendications

1. Système thérapeutique transdermique (TTS) constitué par une couche arrière imperméable aux principes actifs, au moins une couche matricielle à base de polyacrylate renfermant le fentanyl et une couche de protection amovible avant l'utilisation, **caractérisé en ce que**, le polyacrylate est autocollant, exempt de groupes carboxyle, préparé uniquement à partir de monomères esters d'alcools présentant de 1 à 8 atomes de carbone, qui renferment éventuellement un groupe hydroxyle, et d'acide acrylique et/ou acide méthacrylique et éventuellement de jusqu'à 50 % en masse d'acétate de vinyle, présentant une saturation de la solubilité pour le fentanyl comprise entre 3 et 20 % en masse, et **en ce que** les couches renfermant le principe actif présentent une teneur au moins 5 % en masse en fentanyl incorporé et qu'au moins 80 % en masse sont présents sous forme de dispersion moléculaire.

2. TTS selon la revendication 1, **caractérisé en ce que** le polyacrylate présente une saturation de la solubilité pour le fentanyl comprise entre 4 et 12 % en masse.

3. TTS selon la revendication 2, **caractérisé en ce que** le polyacrylate présente une saturation de la solubilité pour le fentanyl comprise entre 5 et 10 % en masse.

4. TTS selon la revendication 1, **caractérisé en ce que** le polyacrylate ne renferme pas de groupes hydroxyle.

5. TTS selon l'une des revendications 1 à 3, **caractérisé en ce que** le mélange de monomères servant de base au polyacrylate présente une teneur jusqu'à 20 % en acrylate et/ou méthacrylate de 2-hydroxyéthyle en tant que monomères présentant des groupes hydroxyle libres.

6. TTS selon l'une des revendications 1 à 3 et 5, **caractérisé en ce que** les chaînes polymères dans le polyacrylate avec des groupes hydroxyle libres sont réticulées par des cations multifonctionnels ou substances réactives.

7. TTS selon la revendication 6, **caractérisé en ce que** la réticulation est réalisée au moyen de Al³⁺, Ti⁴⁺ ou la mélamine.

8. TTS selon l'une des revendications 1 à 7, **caractérisé en ce qu'**il présente une membrane de contrôle en tant qu'une couche supplémentaire.

9. TTS selon la revendication 8, **caractérisé qu'**il présente de plus, côté peau, une couche autocollante disposée sur la membrane de contrôle, pour la fixation à la peau.

10. TTS selon la revendication 8 ou 9, **caractérisé en ce que** la membrane de contrôle est en un copolymère éthylène-acétate de vinyle, de façon appropriée présentant un taux d'acétate de vinyle jusqu'à 25 % en masse, ou en une feuille microporeuse à base de polyéthylène ou de polypropylène et présente de façon appropriée une épaisseur comprise entre 25 et 100, de préférence entre 40 et 100 µm.

11. TTS selon l'une des revendications 1 à 10, **caractérisé en ce que** les couches renfermant le fentanyl renferment de plus des substances qui améliorent le taux de perméation à travers la peau humaine, en particulier des glycols et/ou telles qui appartiennent au groupe des acides gras, des esters d'acides gras, des alcools gras ou des esters de la glycérine.

12. TTS selon l'une des revendications 1 à 11, **caractérisé en ce que** les couches renfermant le fentanyl renferment des substances qui réduisent la solubilité du fentanyl dans ces couches.

13. TTS selon la revendication 12, **caractérisé en ce que** les substances qui réduisent la solubilité à température ambiante sont des hydrocarbures liquides, en particulier le dioctylcyclohexane ou la paraffine liquide, les résines à base d'hydrocarbures, en particulier les résines du polypinène ou le polyéthylèneglycol ou la glycérine.
